# EUROPEAN PATENT APPLICATION

(11) **EP 4 230 717 A1**
(43) Date of publication of application: **23.08.2023**
(21) Application number: 21880220.5
(22) Date of filing: 15.10.2021
(51) Int. Cl.: C12M 1/26, B04B 11/04, C12M 1/00

(54) **TOOL FOR PIPETTE AND KIT FOR CENTRIFUGATION**

(30) Priority: 16.10.2020 JP 2020174831
(71) Applicant: Nipro Corporation, Osaka-shi, Osaka 531-8510 (JP)
(72) Inventor: YOSHIKAWA, Yoshihiro, Osaka-shi, Osaka 531-8510 (JP); TOYOSHIMA, Masayuki, Osaka-shi, Osaka 531-8510 (JP); NAKAMURA, Ryosuke, Osaka-shi, Osaka 531-8510 (JP); ABE, Kazunari, Osaka-shi, Osaka 531-8510 (JP)
(74) Representative: Kuhnen & Wacker Patent- und Rechtsanwaltsbüro PartG mbB
(86) International application number: PCT/JP2021/038239
(87) International publication number: WO 2022/080485

(57) **Abstract**

One aspect of the disclosure provides a pipette tool, comprising a shield plate that includes a first major surface; and a pipe member that includes a first end portion, a second end portion, and a surrounding wall defining an internal space, wherein an end surface of the first end portion of the pipe member is fixed to the first major surface of the shield plate; the pipe member includes in the surrounding wall in the first end portion a first opening communicating with the internal space and includes in an end surface of the second end portion a second opening communicating with the internal space, an outer periphery of the end surface of the first end portion of the pipe member is inward to an outer periphery of the first major surface of the shield plate in the view from a direction of a major axis of the pipe member.

## Description

### TECHNICAL FIELD

The present invention relates to a pipette tool and a kit used in centrifugation. More specifically, the present invention relates to a pipette tool and a kit used in centrifugation, provided with a structure suitable for aspirating a solution (supernatant) from the solution including cells precipitated by centrifugation of a cell suspension while preventing the precipitated cells from floating.

### BACKGROUND ART

Technologies for culturing animal cells, such as human cells, have been developed. Immortalized cells or primary cultured cells are utilized in basic research and trials such as drug developmental non-clinical cases. Induced pluripotent stem cells or mesenchymal stem cells are used to manufacture regenerative medical products.

In tests such as screening tests in which cultured cells are utilized, multi-well microplates are used to culture and propagate cells. The culture medium in the microplates is exchanged with a pipette to aspirate the culture medium from the wells containing cultured cells and then to put fresh culture medium in the wells. The culture cells are sometimes removed together with the culture medium in aspirating the medium with the pipette. A pipette tool with a filter installed to its opening for aspiration was developed to prevent cultured cells from being aspirated (Patent Literature 1).

Regenerative medical products include those produced with cultured allogeneic cells derived from a different individual in the same species or cultured autologous cells derived from the same individual. Cells are repeatedly cultured and propagated until reaching a sufficient number of cells to produce a cultured cells-regenerative medical product. Repeating the culture and propagation of cells requires a process of passaging cells. The cell passage process comprises steps: harvesting a cell suspension containing cultured and propagated cells in a centrifuge tube; subjecting the harvested cell suspension to centrifugation to separate it into a cell pellet and a solution (supernatant); discarding the separated solution; and seeding the remaining cell pellet again in a culture vessel with a fresh culture medium.

### CITATION LIST

Patent Literature 1: JP 2016-185127 A

### SUMMARY

### TECHNICAL PROBLEM

After a cell suspension is centrifuged to separate into a solution and a cell pellet, aspirating the solution with a pipette tool may cause a liquid flow, which renders the cell pellet float. If the floated cells are aspirated together with the solution, the rate of collecting cells decreases. The object of the present invention is to collect centrifuged cells efficiently and stably.

### SOLUTION TO PROBLEM

The present invention relates to a pipette tool and kit used in centrifugation described below.
[Item 1] A pipette tool comprising a shield plate that includes a first major surface; and a pipe member that includes a first end portion, a second end portion, and a surrounding wall defining an internal space, wherein the pipe member is fixed at an end surface of the first end portion to the first major surface of the shield plate; includes in the surrounding wall in the first end portion a first opening communicating with the internal space; and includes in an end surface of the second end portion a second opening communicating with the internal space, an outer periphery of the end surface of the first end portion of the pipe member is inward to an outer periphery of the first major surface of the shield plate in the view from a direction of a major axis of the pipe member.
[Item 2] The pipette tool according to item 1, wherein the first opening is in contact with the first major surface of the shield plate.
[Item 3] The pipette tool according to item 1 or 2, wherein the pipe member includes in the surrounding wall in the first end portion at least two first openings communicating with the internal space.
[Item 4] The pipette tool according to any one of items 1 to 3, wherein an angle between the major axis of the pipe member and the first major surface of the shield plate is 70° to 90°.
[Item 5] The pipette tool according to any one of items 1 to 4, wherein the end surface of the first end portion of the pipe member is fixed at a center of the first major surface of the shield plate.
[Item 6] The pipette tool according to any one of items 1 to 5, wherein the pipe member is cylindrical columnar, elliptical columnar, or polygonal columnar in shape.
[Item 7] The pipette tool according to any one of items 1 to 6, wherein the shield plate includes a second major surface facing the first major surface, an outer periphery of the second major surface of the shield plate is inward to the outer periphery of the first major surface of the shield plate in the view from a direction orthogonal to the first major surface of the shield plate.
[Item 8] The pipette tool according to any one of items 1 to 6, wherein the shield plate includes a second major surface facing the first major surface; the shield plate is tapered in diameter from the first major surface to the second major surface.
[Item 9] The pipette tool according to any one of items 1 to 8, wherein the first major surface and the second major surface of the shield plate are circular, respectively.
[Item 10] A kit used in centrifugation containing a centrifuge tube that comprises a main tube body; and a pipette tool that comprises a shield plate including a first major surface and a pipe member including a first end portion, a second end portion, and a surrounding wall defining an internal space, wherein the pipe member of the pipette tool is fixed at an end surface of the first end portion to the first major surface of the shield plate; includes in the surrounding wall in the first end portion a first opening communicating with the internal space; and includes in an end surface of the second end portion a second opening communicating with the internal space, an outer periphery of the end surface of the first end portion of the pipe member is inward to an outer periphery of the first major surface of the shield plate in the view from a direction of a major axis of the pipe member, and a maximum length of the outer periphery of the first major surface of the shield plate of the pipette tool is shorter than an inner diameter of the main tube body.
[Item 11] The kit according to item 10, wherein the main tube body of the centrifuge tube includes a closed-end portion, the closed-end portion is tapered in diameter from an upper region thereof to a tip region thereof, and in a case where a cross-section orthogonal to a major axis of the centrifuge tube is defined at a position where the shield plate contacts an inner surface of the closed-end portion, a shape of an outer periphery of the shield plate at the cross-section is the same as a shape of an inner periphery of the centrifuge tube at the cross-section.
[Item 12] The kit according to item 10 or 11, wherein the shield plate of the pipette tool includes a second major surface facing the first major surface, is tapered in diameter from the first major surface to the second major surface, the main tube body of the centrifuge tube includes a closed-end portion, the closed-end portion is tapered in diameter from an upper region thereof to a tip region thereof, and a taper angle α of the shield plate is within 15° in difference from a taper angle β of the closed-end portion of the main tube body.

### EFFECT

A pipette tool according to an aspect of the present invention allows for suppressing the floating of precipitated cells caused by solution flow in aspirating the solution separated by the centrifugation, resulting in an efficient collection of the centrifuged cells. Further, it may suppress variations in operations by user(s), resulting in a stable collection of cells.

### BRIEF DESCRIPTION OF DRAWINGS

FIG. 1 is a perspective diagram of a pipette tool according to Embodiment 1.
FIG. 2 is a front view diagram of the pipette tool according to Embodiment 1.
FIG. 3 is a plan view diagram of the pipette tool according to Embodiment 1.
FIG. 4 is a bottom view diagram of the pipette tool according to Embodiment 1.
FIG. 5 is a cross-sectional diagram along the A-A line in FIG. 2.
FIG. 6 is a front view diagram of a pipette tool according to Embodiment 2.
FIG. 7 is a cross-sectional diagram along the A-A line in FIG. 6.
FIG. 8 is a front view diagram of a pipette tool according to Embodiment 3.
FIG. 9 is a cross-sectional diagram along the A-A line in FIG. 8.
FIG. 10 is a perspective diagram of a pipette tool according to Embodiment 4.
FIG. 11 is a perspective diagram of a conventional pipette tool.
FIG. 12 is a front view diagram of a kit used in centrifugation according to Embodiment 5.

### DESCRIPTION OF EMBODIMENTS

Embodiments according to aspects of the present invention are described below with reference to the accompanied drawings, but they are representative examples according to the present invention and do not limit in any way the invention recited in the claims.

### EMBODIMENT 1

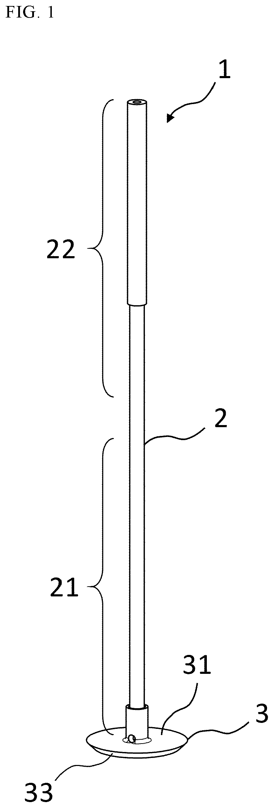

FIG. 1 is a perspective diagram of a pipette tool 1 according to an embodiment of the present invention ("Embodiment 1"). The pipette tool 1 according to Embodiment 1 is provided with a structure suitable for aspirating a solution (supernatant) such as a culture medium, separated by centrifuging a cell suspension in a centrifuge tube, while preventing the precipitated cells from floating in the centrifuge tube. The pipette tool 1 comprises a pipe member 2 and a shield plate 3 attached to the pipe member 2. The pipe member 2 is fixed at an end surface of its first end portion 21 to a first major surface 31 of the shield plate 3.

The pipe member 2 includes the first end portion 21 and a second end portion 22. The first end portion 21 is a portion extending from the end surface of the first end portion 21 toward the second end portion 22, the portion being in length no longer than half the shortest distance between the end surface of the first end portion 21 and an end surface of the second end portion 22. The second end portion 22 is a portion extending from the end surface of the second end portion 22 toward the first end portion 21, the portion being in length no longer than half the shortest distance between the end surface of the first end portion 21 and the end surface of the second end portion 22. The pipette tool 1 according to Embodiment 1 may be fitted with a dispensing implement such as a pipettor or aspirator to the second end portion 22. The pipette tool 1 fitted with a dispensing or aspirating implement can be used to aspirate a solution (supernatant) on a cell pellet in a centrifuge tube 6 after centrifugation.

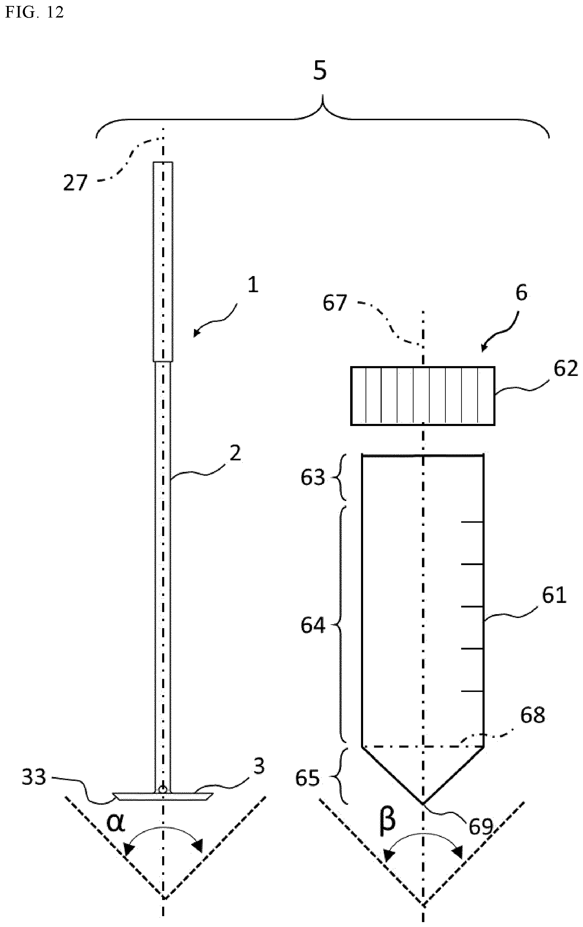

The pipe member 2 according to the present invention has a length and an outer diameter (thickness) such that the shield plate 3 can be moved inside a main tube body 61 of the centrifuge tube 6 along a direction of a major axis of the main tube body 61 (see FIG. 12). The length of the pipe member 2 may be longer than the length of the main tube body 61 along the major axis direction, e.g., 50 to 400 mm, 75 to 300 mm, or 100 to 200 mm. The outer diameter of the pipe member 2 may be smaller than an inner diameter of the main tube body 61, e.g., 3 to 15ϕ mm, 3 to 10ϕ mm, or 3 to 8ϕ mm. The inner diameter of the pipe member 2 may be, for example, 1.5 to 13,5ϕ mm, 1.5 to 8.5ϕ mm, or 1.5 to 6.5ϕ mm. The inner diameter of the main tube body 61 of the centrifuge tube 6 refers to a maximum length of an inner periphery of a surrounding wall of the main body portion 64 of the main tube body 61 at a cross-section of the main body portion 64 of the main tube body 61, the cross-section being orthogonal to the major axis 67. The outer diameter of the pipe member 2 of the pipette tool 1 refers to a maximum length of an outer periphery of a surrounding wall 23 of the pipe member 2 at a cross-section of the pipe member 2 orthogonal to the major axis 27 of the pipe member 2. The inner diameter of the pipe member 2 of the pipette tool 1 is a maximum length of an inner periphery of the surrounding wall 23 of the pipe member 2 at the cross-section of the pipe member 2 orthogonal to the major axis 27 of the pipe member 2. Herein, when the shape is circular, the maximum length is its diameter. When the shape is elliptical, the maximum length is the length of its major axis. When the shape is polygonal (e.g., hexagonal, octagonal, and decagonal), the maximum length is the diameter of a circumscribed circle thereto.

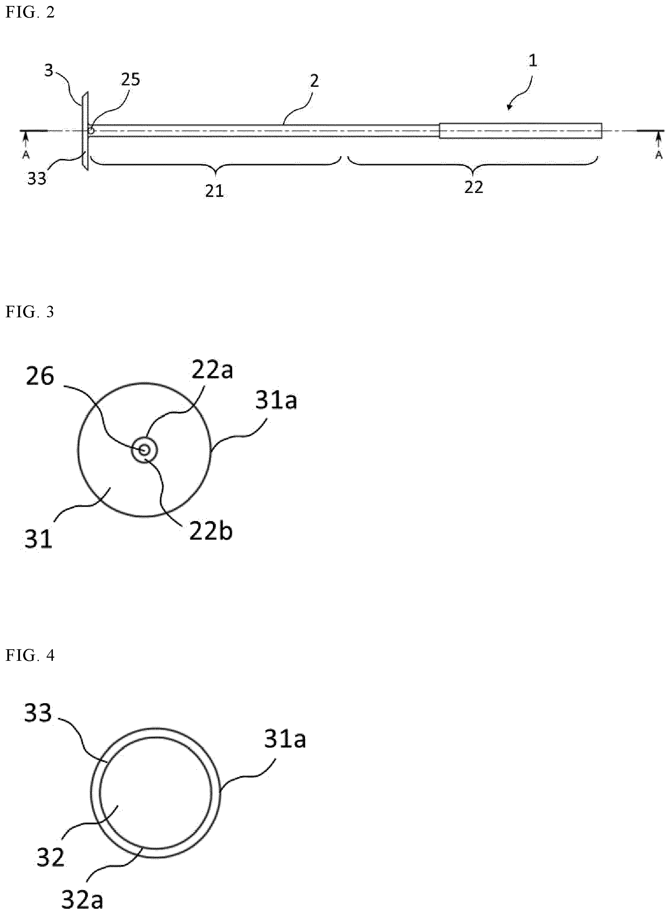

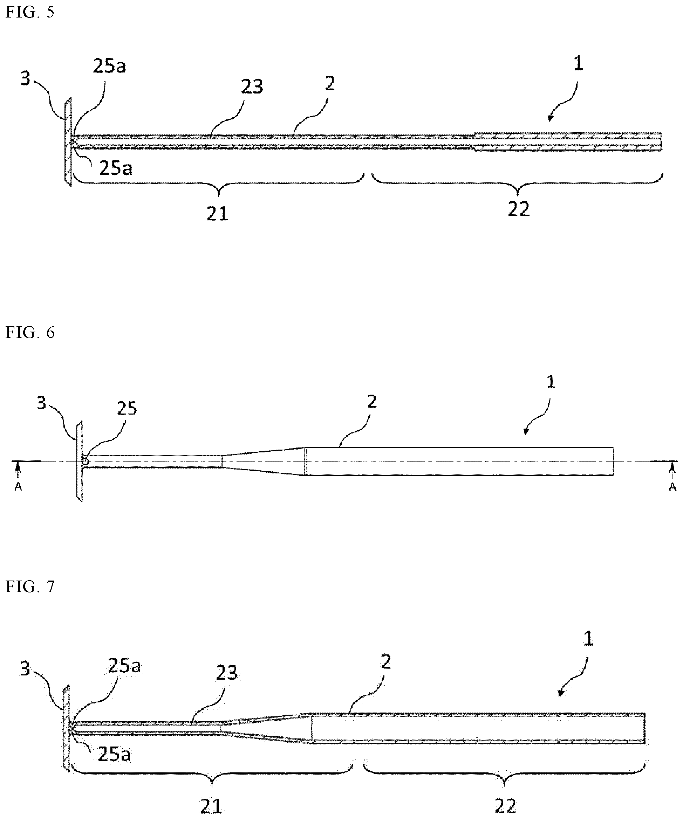

The pipe member 2 according to Embodiment 1 includes two first openings 25 in the peripheral wall of the first end portion 21 (see FIG. 2). FIG. 2 illustrates one of the two first openings 25. The other first opening 25 is provided on the back side, which is not illustrated in the drawing. FIG. 3 is a plan view diagram of the pipette tool according to Embodiment 1. The pipe member 2 includes a second opening 26 in an end surface 22b of the second end portion 22, the opening communicating with the internal space of the pipe member 2. As illustrated in FIG. 5, the two first openings 25 in Embodiment 1 communicate with the internal space of the pipe member 2 defined by the surrounding wall 23. The internal space of the pipe member 2 communicates with the second opening 26 in the second end portion of the pipe member 2.

To aspirate, from the centrifuge tube 6 containing a centrifuged cell pellet and a solution (supernatant) thereon, the solution with the pipette tool 1 according to Embodiment 1, the pipette tool 1 is moved inside the centrifuge tube 6 through its open-end portion 63 to its closed-end portion 65 along the major axis 67. The shield plate 3 of the pipette tool 1 is immersed in the solution (supernatant) and brought close to the cell pellet. Through the first openings 25 of the pipette tool 1 located in the solution, the solution is aspirated by the suction force, for example, induced by a dispensing or aspirating implement fitted to the second end portion 22 of the pipette member 2. The aspirated solution is to go through the internal space of the pipe member 2 communicating with the first openings 25 and discharged through the second opening 26 communicating with the internal space. The aspiration of the solution causes a liquid flow in the solution in the centrifuge tube 6. The shield plate 3, located close to the cell pellet, can prevent cells of the cell pellet from floating caused by the liquid flow. Thus, the shield plate 3 of the pipette tool 1 can prevent cells from floating out of the cell pellet in aspirating the solution, suppressing the cells being aspirated with the solution, resulting in a stable and efficient collection of cells.

When the pipe member 2 of the pipette tool 1 has enough internal space to hold the solution, holding in the internal space of the pipette tool 1 the solution of the centrifuge tube and bringing the pipette tool 1 out from the centrifuge tube would result in the discharge of the solution from the centrifuge tube. The internal space of the pipe member 2 enlarges by increasing the inner diameter of the pipe member 2 and/or the length of the pipe member 2.

The pipe member 2 according to Embodiment 1 comprises, in the surrounding wall 23 of the first end portion 21, two first openings 25 communicating with the internal space. The number of the first openings 25 according to the present invention may be, for example, one, two, three, four, or five. The two first openings 25 according to Embodiment 1 are opposite each other. In the present invention, a plurality of the first openings 25 are provided on the surrounding wall 23 of the first end portion 21, where they are arranged with interval spaces around a central point in the view from the major axis direction of the pipe member 2. For example, the plurality of the first openings 25 are arranged with equal interval spaces around the central point in the view from the major axis direction of the pipe member 2.

The two first openings 25 according to Embodiment 1 are provided in contact with the first major surface 31 of the shield plate 3. The first opening 25 according to the present invention may be provided, for example, at a position where a surface portion of its wall surface 25a closest to the first major surface 31 is distant in a direction extending from the end surface of the first end portion 21 to the end surface of the second end portion 22 with no more than 10%, no more than 5%, or no more than 3% of the shortest length between the end surface of the first end portion 21 and the end surface of the second end portion 22. The first opening 25 according to the present invention is provided at a position where the surface portion of the wall surface 25a closest to the first major surface 31 is a distance of 0 to 20 mm, 0 to 10 mm, or 0 to 5 mm from the first major surface 3 of the shield plate 3.

For example, the first opening 25 may be circular, polygonal (e.g., hexagonal, octagonal, and decagonal), or elliptical in shape. For example, the first opening 25 is 1 to 5 mm, 1.5 to 4 mm, or 2 to 3 mm in maximum length.

As illustrated in FIG. 3, the second end portion of the pipe member 2 includes the end surface 22b, of which an outer periphery 22a is inward to an outer periphery 31a of the first major surface 31 of the shield plate 3 in the view of the first end portion 22 of the pipe member 2 from the major axis direction of the pipe member 2. Further, the first end portion 21 of the pipe member 2 according to Embodiment 1 includes the end surface, of which an outer periphery is inward to the outer periphery 31a of the first major surface 31 of the shield plate 3 in the view of the first end portion 22 of the pipe member 2 from the major axis direction of the pipe member 2.

In Embodiment 1 (see FIG. 12), the angle (acute angle) between the major axis 27 of the pipe member 2 and the first major surface 31 of the shield plate 3 is about 90° (e.g., 85° to 90°). In the present invention, the angle between the major axis 27 of the pipe member 2 and the first major surface 31 of the shield plate 3 may be, for example, 60° to 90°, 70° to 90°, or 80° to 90°. The angle between the major axis 27 of the pipe member and the first major surface 31 of the shield plate 3 may be, for example, 60°, 65°, 70°, 75°, 80°, 85°, or 90°.

The pipe member 2 according to Embodiment 1 is cylindrical columnar in shape. The pipe member 2 according to the present invention may be, for example, polygonal columnar (e.g., hexagonal, octagonal, and decagonal columnar), elliptical columnar, or cylindrical columnar in shape. The pipe member 2 according to the present invention may be tapered in part or in entirety to reduce the diameter from the end surface 22b of the second end portion 22 to the end surface of the first end portion 21.

The surrounding wall 23 of the pipe member 2 may be, for example, 0.5 to 1 mm, 0.6 to 0.9 mm, or 0.7 to 0.8 mm in thickness. In an embodiment, the surrounding wall 23 of the pipe member 2 is constant in thickness from the first end portion 21 to the second end portion 22. In an embodiment, the surrounding wall 23 of the pipe member 2 may be inconstant in thickness from the first end portion 21 to the second end portion 22. In an embodiment, the surrounding wall 23 of the second end part 22 is thicker than the surrounding wall 23 of the first end part 21.

The shield plate 3 according to Embodiment 1 is in shape and size that allows for moving inside the main tube body 61 of the centrifuge tube 6 along the major axis 67 (see FIG. 12). For example, the maximum length of the outer periphery 31a of the first major surface of the shield plate 3 is shorter than the inner diameter of the main tube body 61 of the centrifuge tube 6.

The shield plate 3 according to Embodiment 1 includes the first major surface 31, a second major surface 32 facing the first major surface 31, and a lateral surface 33 connecting the first major surface 31 and the second major surface 32 (see FIGs. 1 and 4). The shield plate 3 may be, for example, 1 to 7 mm, 2 to 5 mm, or 2 to 4 mm in thickness. The shield plate 3 may be, for example, 1 mm, 2 mm, 3 mm, 4 mm, 5 mm, 6 mm, or 7 mm in thickness. In an embodiment, the shield plate 3 is constant in thickness through the shield plate 3 entirely. In an embodiment, the shield plate 3 is inconstant in thickness through the shield plate 3 entirely.

As illustrated in FIG. 4, the shield plate 3 according to Embodiment 1 includes the second major surface, of which an outer periphery 32a is inward to the outer periphery 31a of the first major surface of the shield plate 3 in view of the second major surface 32 from a direction orthogonal to the first major surface 31 of the shield plate 3. As illustrated in FIG. 1, the shield plate 3 according to Embodiment 1 is cylindrical columnar in shape, with its outer periphery gradually decreasing from the first major surface 31 to the second major surface 32. The first major surface 31 and second major surface 32 of the shield plate 3 are circular, respectively. The shield plate 3 according to the invention may be, for example, polygonal columnar (e.g., hexagonal, octagonal, and decagonal columnar), elliptical columnar, or cylindrical columnar in shape. The first major surface 31 and second major surface 32 of the shield plate 3 according to the invention may be, for example, polygonal (e.g., hexagonal, octagonal, and decagonal) or elliptical in shape, respectively.

In Embodiment 1, the shield plate 3 is tapered in diameter from the first major surface 31 to the second major surface 32. The shield plate 3 comprises the lateral surface 33 tapered from the first major surface to the second major surface. A taper angle α of the shield plate 3 or taper angle α of the lateral surface 33 of the shield plate 3 (see FIG. 12) may be, for example, 20° to 160°, 40° to 140°, or 60° to 120°. The angle α may be, for example, 20°, 30°, 40°, 50°, 60°, 70°, 80°, 90°, 100°, 110°, 120°, 130°, 140°, 150°, or 160°.

In Embodiment 1, the end surface of the first end portion 21 of the pipe member 2 is fixed to the first major surface 31 of the shield plate 3 at the geometric center (see FIG. 3). For example, the pipe member 2 according to the present invention may be fixed to the first major surface 31 at the center. The center of the first major surface 31 refers to an area where the distance is no more than 10% of the maximum length of the first major surface from the geometric center of the first major surface 31 in shape.

The shield plate 3 according to the present invention is made, for example, from transparency, semitransparency, or opaque plastic material, rubber, or glass. The shield plate 3 is made, for example, from metal. The plastic material includes, for example, plastics customarily used in the pharmaceutical or research field, such as polystyrene, polycarbonate, polyethylene, polypropylene, and polyethylene terephthalate. The rubber includes, for example, rubber customarily used in the pharmaceutical or research field, such as butyl rubber, isoprene rubber, butadiene rubber, and silicone rubber. The glass or metal includes, for example, glass or metal customarily used in the pharmaceutical or research fields.

The pipette tool 1 according to the present invention can be produced according to known methods. For example, the pipette tool 1 can be produced by injection molding. The pipette tool 1 can be produced as one piece or an assembly of two or more members. When the pipette tool 1 is produced by assembling two or more members, the two or more members may be made from the same material or different materials. The pipette tool 1 according to Embodiment 1 can be produced by integrally molding the pipe member 2 and the shield plate 3.

### EMBODIMENT 2

FIG. 6 illustrates a front view diagram of a pipette tool 1 according to another embodiment of the present invention ("Embodiment 2"). Embodiment 2 is described below with reference to FIG. 6. The description focuses on different features from Embodiment 1, and the description of the similar features is omitted.

In Embodiment 1, the pipe member 2 of the pipette tool 1 is approximately constant in outer diameter from the first end portion 21 to the second end portion 22 (see FIG. 2). In Embodiment 2 as illustrated in FIG. 6, a pipe member 2 of the pipette tool 1 is tapered in outer diameter from an end surface of a second end portion 22 to an end surface of a first end portion 21 in between the end surface of the first end portion 21 and the end surface of the second end portion 22.

In Embodiment 1, the pipe member 2 of the pipette tool 1 is approximately constant in inner diameter from the first end portion 21 to the second end portion 22 (see FIG. 5). In Embodiment 2 as illustrated in FIG. 7, the pipe member 2 of the pipette tool 1 is tapered in inner diameter from the end surface of the second end portion 22 to the end surface of the first end portion 21 in between the end surface of the first end portion 21 and the end surface of the second end portion 22.

### EMBODIMENT 3

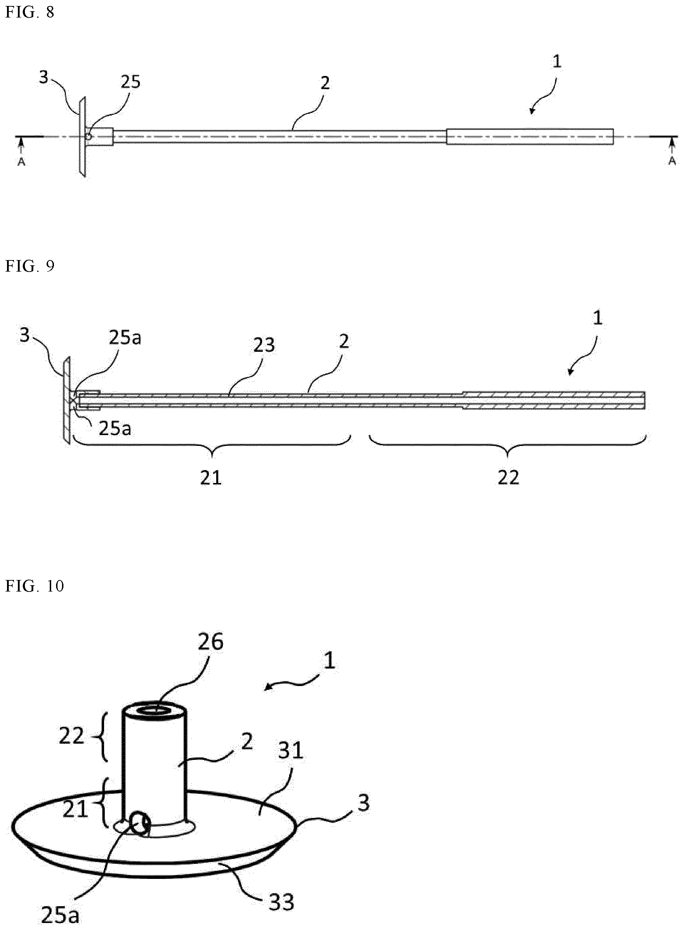

FIG. 8 illustrates a front view diagram of a pipette tool 1 according to another embodiment of the present invention ("Embodiment 3"). Embodiment 3 is described below with reference to FIG. 8. The description focuses on different features from Embodiment 1, and the description of the similar features is omitted.

In Embodiment 1, the pipette tool 1 can be produced by integrally molding the pipe member 2 and the shield plate 3 (see FIG. 1). In Embodiment 3 as illustrated in FIG. 9, the pipette tool 1 is produced by assembling two members, the first member primarily constituting a pipe member 2 and the second member primarily constituting a shield plate 3. The first and second members may be made from the same material or different materials, respectively.

### EMBODIMENT 4

FIG. 10 illustrates a perspective diagram of a pipette tool 1 according to another embodiment of the present invention ("Embodiment 4"). Embodiment 4 is described below with reference to FIG. 10. The description focuses on different features from Embodiment 1, and the description of the similar features is omitted.

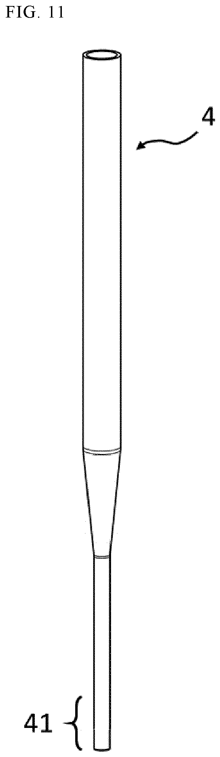

In Embodiment 1, the pipe member 2 of the pipette tool 1 is longer than the length of the centrifuge tube along the major axis direction. The pipette tool 1 according to Embodiment 1 may be fitted with a dispensing or aspirating implement such as a pipettor or aspirator to the second end portion 22 of the pipe member 2. In Embodiment 4, a pipe member 2 of the pipette tool 1 is sufficiently shorter than the length of the centrifuge tube along the direction of its major axis. The pipette tool 1 according to Embodiment 4 may be fitted via its second end portion 22 to a tip portion 41 of a conventional pipette 4 (see FIG. 11). The pipette tool 1 according to Embodiment 4 fitted to the conventional pipette 4 can be used in the same manner described herein for the pipette tool 1 according to Embodiments 1 to 3.

### EMBODIMENT 5

FIG. 12 illustrates a front view diagram of a kit 5 used in centrifugation according to another embodiment of the present invention ("Embodiment 5"). The kit 5 used in centrifugation contains a pipette tool 1 and a centrifuge tube 6, disclosed herein. The kit 5 used in centrifugation may contain a plurality of the pipette tools 1 and a plurality of the centrifuge tubes 6.

The centrifuge tube 6 comprises a main tube body 61 and a screw cap 62. The main tube body 61 comprises a surrounding wall defining an internal space. The surrounding wall of the main tube body 61 includes an open-end portion 63, a main body portion 64, and a closed-end portion 65. An inner periphery of a cross-section of the main tube body 61 orthogonal to a major axis 67 of the main tube body 61 is, for example, circular, elliptical, or polygonal (e.g., hexagonal, octagonal, and decagonal). The main tube body 61 according to the present invention can hold, for example, 10 to 250 ml, 20 to 200 ml, or 50 to 150 ml of solution in the internal space. The main tube body 61 may be, for example, 25 to 350 mm, 50 to 250 mm, or 75 to 150 mm in length along the major axis direction. An inner diameter of the main tube body 61 may be 10 to 100ϕ mm, 20 to 80ϕ mm, or 30 to 70ϕ mm. The surrounding wall of the main tube body 61 may be, for example, 0.5 to 3 mm, 1 to 2.5 mm, or 1.5 to 2 mm in thickness. In an embodiment, the surrounding wall of the main tube body 61 is constant in thickness from the open-end portion 63 to the closed-end portion 65. In an embodiment, the surrounding wall of the main tube body 61 is inconstant in thickness from the open-end portion 63 to the closed-end portion 65.

The open-end portion 63 of the main tube body 61 according to Embodiment 5 is provided with a structure for receiving a screw cap 62. The centrifuge tube 6 is opened and closed using the screw cap 62. The centrifuge tube 6 according to the present invention may be opened and closed with a hinge cap or a screw cap.

In Embodiment 5, a shield plate 3 of the pipette tool 1 is in shape and size that allows for moving inside the main tube body 61 of the centrifuge tube 6 along the major axis 67. The shield plate 3 of the pipette tool 1 according to Embodiment 5 includes a first major surface 31 of which an outer periphery is smaller in maximum length than an inner diameter of the main tube body 61. The maximum length of the outer periphery 31a of the first major surface 31 of the shield plate 3 is also shorter than the inner diameter of the main body portion 64 of the main tube body 61. For example, the maximum length of the outer periphery 31a of the first major surface of the shield plate 3 is shorter than the main tube body 61 or the inner diameter of the main body portion 64 by 5 to 60ϕ mm, 10 to 50ϕ mm, or 15 to 40ϕ mm. When the centrifuge tube 6 is provided with a hinge cap, the maximum length of the outer periphery 31a of the first major surface of the shield plate 3 is, for example, shorter than an inner diameter of an opening part of the hinge cap. The opening part of the hinge cap includes a surrounding wall defining the opening. The inner diameter of the opening part of the hinge cap refers to a maximum length of the inner periphery of the surrounding wall defining an opening at a cross-section orthogonal to a central axis of an opening face in the opening part.

In Embodiment 5, the first major surface 31 and the second major surface 32 of the shield plate 3 of the pipette tool 1 are circular in the shape of their outer peripheries, which are the same shape that the inner periphery of the centrifuge tube 6 is at a cross-section orthogonal to the major axis of the centrifuge tube 6. The shapes of the outer peripheries of the first major surface 31 and the second major surface 32 of the shield plate 3 are, for example, circular, elliptical, or polygonal (e.g., hexagonal, octagonal, and decagonal). In an embodiment, the outer peripheries of the first major surface 31 and second major surface 32 of the shield plate 3 may be or may not be the same in shape as the inner periphery of the main tube body 61 at the cross-section orthologue to the major axis of the centrifuge tube 6.

For example, the length of the pipette tool 1 along the direction of its major axis is longer than the length of centrifuge tube 6 along a direction of its major axis by 10 to 100 mm, 15 to 80 mm, or 20 to 60 mm.

As illustrated in FIG. 12, the shield plate 31 of the pipette tool 1 according to Embodiment 5 comprises the first major surface 31 and the second major surface 32 facing the first major surface 31 and is tapered in diameter from the first major surface 31 to the second major surface 32. The shield plate 31 includes a lateral surface 33 connecting the first major surface 31 and the second major surface 32. The lateral surface 33 is tapered in diameter from the first major surface 31 to the second major surface 32. A taper angle α of the shield plate 3 or taper angle α of the lateral surface 33 of the shield plate 3 may be, for example, 20° to 160°, 40° to 140°, or 60° to 120°. The angle α may be, for example, 20°, 30°, 40°, 50°, 60°, 70°, 80°, 90°, 100°, 110°, 120°, 130°, 140°, 150°, or 160°.

The closed-end portion 65 of the centrifuge tube 6 according to Embodiment 5 is tapered in diameter from an upper region 68 to a tip region 69. A taper angle β of the closed-end portion 65 of the main tube body 61 may be, for example, 20° to 160°, 40° to 140°, or 60° to 120°. The angle α may be, for example, 20°, 30°, 40°, 50°, 60°, 70°, 80°, 90°, 100°, 110°, 120°, 130°, 140°, 150°, or 160°.

In Embodiment 5, the taper angle α of the shield plate 3 of the pipette tool 1 is equal to the taper angle β of the closed-end portion 65 of the main tube body 61. In the kit according to the present invention, the taper angle α may be equal to or different from the taper angle β. When the taper angle α is not equal to the taper angle β, the difference between the taper angle α and the taper angle β may be, for example, within 15°, within 10°, within 5°, or within 3°.

In Embodiment 5, to aspirate a solution (supernatant) from the centrifugate tube 6 containing the solution in its upper part and a cell pellet (around the tip region 69 of the closed-end portion 65 of centrifuge tube 6), the pipette tool 1 is operated as described below. First, put the pipette tool 1 into the centrifuge tube 6 through the open-end portion 63 and move it along the major axis 67 from the open-end portion 63 to the closed-end portion 65. Immerse the shield plate 3 of the pipette tool 1 in the solution (supernatant) and then move it to approach the cell pellet until being in contact with the inner surface of the closed-end portion 65 of the centrifuge tube 6. The shield plate 3 of the pipette tool 1 is retained at the position (referred to as "contact position" or "contacting position") and cannot move further forward to the tip region 69 of the closed-end part 65. In aspirating the solution (supernatant) through a first opening 25 of the pipette tool 1, the shield plate 3, positioned between the first opening 25 and the cell pellet, facilitates aspirating the solution above there and preventing the cell pellet below there from aspirating the solution together. Thus, the aspiration of the cell pellet can be suppressed. Retaining the shield plate 3 of the pipette tool 1 at the position where it contacts the inner surface of the closed-end portion 65 can suppress variations in the operations by user(s), especially the positioning of the shield plate 3 in aspirating the solution, resulting in a more stable collection of cells.

Furthermore, the shield plate 3 preferably shields almost the entire space on the side of the tip region 69 of centrifuge tube 6 when the shield plate 3 is in contact with the inner surface of centrifuge tube 6. In other words, when a cross-section is defined that passes through the position where the inner surface of the closed-end portion 65 of the centrifuge tube 6 contacts the shield plate 3 and is orthogonal to the major axis 67 of the centrifuge tube 6, the shape of an inner periphery of the centrifuge tube 6 at the cross-section is preferably the same as the shape of an outer periphery of the shield plate 3 at the cross-section. This allows the shield plate 3 to shield the space below the contacting position, *i.e*., the space near the tip region 69 of the closed-end portion 65 where the cell pellet is contained. As a result, the cell pellet in the space below the shield plate 3 can be effectively suppressed from being aspirated together with the solution (supernatant) when the solution is aspirated through the first opening 25 of the pipette tool 1. Moreover, when the difference between the taper angle β of the closed-end portion 65 of the main tube body 61 and the taper angle α of the shield plate 3 of the pipette tool 1 is within 15°, preferably within 10°, within 5°, or within 3°, especially when the taper angle β is equal to the taper angle α, an area of the lateral surface of the shield plate 3 in contact with the inner surface of the centrifuge tube 6 increases. Thus, that can suppress variations in the operations by user(s), especially the positioning of the shield plate 3 in aspirating the solution, resulting in a stable collection of cells.

For example, a main tube body 61 or a cap of the centrifuge tube 6 according to the present invention is made from transparency, semitransparency, or opaque plastic material. Plastic materials include plastics customarily used in the pharmaceutical or research fields, such as polystyrene, polycarbonate, polyethylene, polypropylene, and polyethylene terephthalate. The main tube body 61 and the cap may be made from different materials or the same material.

Known methods can produce the centrifuge tube according to the present invention. The centrifuge tube can be produced, for example, by injection molding.

### EXAMPLE 1

A cell suspension containing cultured mammalian cells was prepared. The number of cells in the cell suspension of 80 ml was counted. As a result, the cell suspension contained approximately 1.0 × 10⁷ cells in 80 ml. The cell suspension of 80 ml was dispensed into a centrifuge tube and then subjected to centrifugation to separate into a cell pellet and solution (supernatant). A pipette tool 1 according to Embodiment 1 was put into the centrifuge tube through its open-end portion and moved so that a shield plate of the pipette tool was positioned 2 to 3 mm ("non-contact position") above the position ("contact position") where the shield plate of the pipette tool was in contact with an inner surface of a closed-end portion of the centrifuge tube. At the non-contact position, the solution (supernatant) was aspirated with the pipette tool. After aspiration, the remaining cells in the centrifuge tube were collected, and the number of the cells was counted.

### COMPARATIVE EXAMPLES

The test was conducted substantially the same as in Example 1, except for substituting a conventional pipette for the pipette tool.

### EXAMPLE 2

The test was conducted substantially the same as in Example 1, except for changing the position where the solution (supernatant) was aspirated with the pipette tool from the non-contact position to the contact position.

The cell collection rate (%) was calculated by dividing the number of collected cells by the number of cells in the cell suspension of 80 ml (1.0 × 10⁷) before centrifugation. Table 1 shows the results.

**[Table 1]**

| | Cell Collection Rate [%] | | | Ave. | STDEV |
|---|---|---|---|---|---|
| | N1 | N2 | N3 | | |
| Comparative Example | 64.45 | 77.83 | 92.74 | 78.34 | 14.15 |
| Example 1 | 88.00 | 91.74 | 103.07 | 94.27 | 7.84 |
| Example 2 | 90.30 | 96.71 | 94.33 | 93.78 | 3.24 |

As seen from Table 1, using the pipette tool according to the present invention yielded over 90% of cell collection rates on average (Examples 1 and 2). The average rates of collecting cells with the pipette tool were superior to the average cell collection rate with the conventional pipette (Comparative Example). The result suggests that the pipette tool according to the present invention enables an efficient collection of cells after centrifugation.

Variation in the cell collection rates with the pipette tool according to the present invention at the non-contact position (Example 1) was smaller than the variation in the cell collection rates with the conventional pipette at the non-contact position (Comparison Example). This result and the above results suggest that the pipette tool according to the present invention enables an efficient and stable collection of cells after centrifugation.

Further, the variation in the cell collection rates with the pipette tool according to the present invention at the contact position (Example 2) was smaller than the variation in the cell collection rates with the pipette tool according to the present invention at the non-contact position (Example 1). The result suggests that using the pipette tool according to the present invention at the contact position enables an efficient and more stable collection of cells after centrifugation.

### EXPLANATION OF REFERENCE SIGNS

- 1: Pipette tool
- 2: Pipe member
- 3: Shield plate
- 4: Conventional pipette
- 5: Kit used in centrifugation
- 6: Centrifuge tube
- 21: First end portion
- 22: Second end portion
- 22a: Outer periphery of second end portion
- 22b: End surface of second end portion
- 23: Surrounding wall
- 25: First opening
- 25a: Wall surface of first opening
- 26: Second opening
- 27: Major axis of pipe member
- 31: First major surface
- 31a: Outer periphery of first major surface
- 32: Second major surface
- 32a: Outer periphery of second major surface
- 33: Lateral surface
- 41: Tip portion
- 61: Main tube body
- 62: Screw cap
- 63: Open-end portion
- 64: Main body portion
- 65: Closed-end portion
- 67: Major axis of centrifuge tube
- 68: Upper region of closed-end portion
- 69: Tip region of closed-end portion

## Claims

1. A pipette tool, comprising
a shield plate that includes a first major surface; and
a pipe member that includes a first end portion, a second end portion, and a surrounding wall defining an internal space,
wherein the pipe member
is fixed at an end surface of the first end portion to the first major surface of the shield plate;
includes in the surrounding wall in the first end portion a first opening communicating with the internal space; and
includes in an end surface of the second end portion a second opening communicating with the internal space,
an outer periphery of the end surface of the first end portion of the pipe member is inward to an outer periphery of the first major surface of the shield plate in the view from a direction of a major axis of the pipe member.

2. The pipette tool according to claim 1, wherein the first opening is in contact with the first major surface of the shield plate.

3. The pipette tool according to claim 1 or 2, wherein the pipe member includes in the surrounding wall in the first end portion at least two first openings communicating with the internal space.

4. The pipette tool according to any one of claims 1 to 3, wherein an angle between the major axis of the pipe member and the first major surface of the shield plate is 70° to 90°.

5. The pipette tool according to any one of claims 1 to 4, wherein the end surface of the first end portion of the pipe member is fixed at a center of the first major surface of the shield plate.

6. The pipette tool according to any one of claims 1 to 5, wherein the pipe member is cylindrical columnar, elliptical columnar, or polygonal columnar in shape.

7. The pipette tool according to any one of claims 1 to 6,
wherein the shield plate includes a second major surface facing the first major surface,
an outer periphery of the second major surface of the shield plate is inward to the outer periphery of the first major surface of the shield plate in the view from a direction orthogonal to the first major surface of the shield plate.

8. The pipette tool according to any one of claims 1 to 6,
wherein the shield plate includes a second major surface facing the first major surface;
the shield plate is tapered in diameter from the first major surface to the second major surface.

9. The pipette tool according to any one of claims 1 to 8, wherein the first major surface and the second major surface of the shield plate are circular, respectively.

10. A kit used in centrifugation containing
a centrifuge tube that comprises a main tube body; and
a pipette tool that comprises a shield plate including a first major surface and a pipe member including a first end portion, a second end portion, and a surrounding wall defining an internal space,
wherein the pipe member of the pipette tool
is fixed at an end surface of the first end portion to the first major surface of the shield plate;
includes in the surrounding wall in the first end portion a first opening communicating with the internal space; and
includes in an end surface of the second end portion a second opening communicating with the internal space,
an outer periphery of the end surface of the first end portion of the pipe member is inward to an outer periphery of the first major surface of the shield plate in the view from a direction of a major axis of the pipe member, and
a maximum length of the outer periphery of the first major surface of the shield plate of the pipette tool is shorter than an inner diameter of the main tube body.

11. The kit according to claim 10,
wherein the main tube body of the centrifuge tube includes a closed-end portion,
the closed-end portion is tapered in diameter from an upper region thereof to a tip region thereof, and
in a case where a cross-section orthogonal to a major axis of the centrifuge tube is defined at a position where the shield plate contacts an inner surface of the closed-end portion, a shape of an outer periphery of the shield plate at the cross-section is the same as a shape of an inner periphery of the centrifuge tube at the cross-section.

12. The kit according to claim 10 or 11,
wherein the shield plate of the pipette tool
includes a second major surface facing the first major surface,
is tapered in diameter from the first major surface to the second major surface,
the main tube body of the centrifuge tube includes a closed-end portion,
the closed-end portion is tapered in diameter from an upper region thereof to a tip region thereof, and
a taper angle α of the shield plate is within 15° in difference from a taper angle β of the closed-end portion of the main tube body.
